# EUROPEAN PATENT SPECIFICATION

(11) **EP 4 381 285 B1**
(45) Date of publication and mention of the grant of the patent: **09.04.2025**
(21) Application number: 22758016.4
(22) Date of filing: 26.07.2022
(51) Int. Cl.: H01G 9/048, H01G 9/07, G01N 27/38, G01N 27/22, G01N 33/18, H01G 11/04, H01G 9/22, H01G 9/28, G01R 27/26, H01G 11/02, H01G 11/08

(54) **ELECTROCHEMICAL BIOFILM SENSOR**
ELEKTROCHEMISCHER BIOFILMSENSOR
CAPTEUR ÉLECTROCHIMIQUE DE BIOFILM

(30) Priority: 05.08.2021 IT 202100021248
(43) Date of publication of application: 12.06.2024
(73) Proprietor: ALVIM Srl, 16161 Genova (GE) (IT)
(72) Inventor: MOLLICA, Alessandro, 16161 Genova (GE) (IT); MOLLICA, Alfonso Maria, 16161 Genova (GE) (IT); PAVANELLO, Giovanni, 16161 Genova (GE) (IT)
(74) Representative: Karaghiosoff, Giorgio Alessandro
(86) International application number: PCT/IB2022/056876
(87) International publication number: WO 2023/012581

(56) References cited:
- WO-A1-2012/149487
- WO-A1-2021/028054
- WO-A2-2009/125354
- US-A1- 2019 105 414
- US-B1- 10 444 180

## Description

The present invention relates to an electrochemical sensor to monitor the proliferation of biofilm in systems in contact with liquids, such as, for instance, pipes, tanks, etc. and wherein the sensor detects the biofilm on its surface.

The term biofilm generally refers to the layer of microorganisms that covers any surface in contact with water or other liquids. In the civil and industrial fields, this biological phenomenon causes numerous problems, ranging from the acceleration of the corrosion processes of metal materials, to the loss of efficiency of the heat exchangers, to the contamination of products and much more. Hence the need to put in place adequate measures aimed at contrasting this phenomenon. To this purpose, it is essential to have a timely indication about the growth of the biofilm, possibly from its earliest stages.

In response to this need, numerous biofilm sensors have been developed over the years, based on different techniques, such as optical measurements, heat exchange, ultrasonics, etc. Among the devices that have shown greater specificity, sensitivity and reliability, there are certainly those based on electrochemical techniques. In particular, some of them, such as e.g. the owner's ALVIM sensors, are able to detect the growth of this bacterial layer by exploiting the alteration, induced by the biofilm, of the kinetics of some evolving reactions on the metal surface of the sensor's working electrode. Such sensors are described, for instance, in the document "Exploiting a new electrochemical sensor for biofilm monitoring and water treatment optimization", G Pavanello et al. - Water research, 2011 - Elsevier.

These devices are basically three-electrode cells, i.e. working electrode, reference electrode and counter-electrode. The working electrode, whereon biofilm growth is monitored, is usually made of a "passive" alloy (e.g. stainless steel or titanium), while the other two auxiliary electrodes are often combined into a single electrode, which performs at the same time the role of counter-electrode and pseudo-reference electrode, normally made up of materials of the "sacrificial anode" type, such as zinc.

In order to detect the aforementioned alterations of the reaction kinetics on the surface of the working electrode induced by the biofilm formation, a constant potential or a constant current are preset to the working electrode. In the first case, as an effect of the gradual formation of the biofilm, the current required varies over time. In the second case, as an effect of the gradual formation of the biofilm, the necessary tension varies over time.

As mentioned above, the aforementioned electrochemical biofilm sensors offer a number of undoubted advantages, however, in certain cases, they may have some limitations.

One of these limitations can be represented by the use of materials of the "sacrificial anode" type, such as zinc, for the counter-electode. In addition to a potential reduction in the life of the biofilm sensor, this can cause reactions with chemicals used in industrial processes, interfering with the operation of the sensor itself, or introducing unwanted metal ions into the process liquid.

Furthermore, in the case of installation in a remote location, any necessary cleaning of the electrodes can be complex and costly. The possibility of "self-cleaning" for this type of sensor would therefore offer an undoubted advantage.

Document WO2012/149487 describes simple galvanic pairs, therefore, two electrodes, possibly differently exposed before the construction of the pair and a third reference electrode to measure the potential of the pair as well as an ammeter to measure the galvanic current. The system aims to see if an "electrogenic", and therefore corrosive (MIC) biofilm is formed, by measuring galvanic currents and/or potentials in pairs formed by electrodes differently exposed in the solution. The setting of a set potential or a set current between the electrodes is not envisaged. Furthermore, the use of electrodes with metal oxide coatings of different metals mixed together, i.e. mixed oxides of different metals, is not envisaged.

In document WO2009/125354 a potentiostat is used to set on a metal sample (defined "working electrode"), whereon the biofilm to be monitored can grow, a desired cathodic potential, a potential, i.e., lower than the free corrosion potential of the electrode, recording the trend of the current supplied. Alternatively, a galvanostat is used to demand the supply of a certain cathodic current from the working electrode, recording the trend of the potential. To set the selected potential on the working electrode, the potentiostat must use a second electrode at constant potential (reference electrode, Ref.) and a third electrode (counter-electrode, C.E.) as generator of the current used by the working electrode. This is to maintain the set potential over time. A similar effect can be obtained with a galvanostat if, after setting a cathodic current on the working electrode, the trend of the potential of the same electrode is observed. It is essentially a classic electrochemical cell with three electrodes (W, Ref, C.E.) used here to set a set cathodic potential on the working electrode via a potentiostat or to demand the supply of a set cathodic current in an intense static configuration. The choice to operate in the cathodic area and to use only some types of material as a working electrode derives from a well-known property of the biofilm: the formation of the biofilm causes an increasing "cathodic depolarization", that is, an increase in the rate of oxygen reduction, on a series of "passive" alloys (e.g. stainless steel, titanium, ...) kept at a constant potential. In other words: the cathodic current observed on the aforementioned alloys used as working electrodes and maintained at a set cathodic potential (i (E, t)) increases over time, thus signaling the growth of the biofilm and allowing an estimate of the entity of the biofilm itself. Analogous effects are obtained with galvanostatic treatment. The document does not provide for the use of electrodes with mixed oxide coatings of different noble metals. Document WO2021/028054 provides for the creation of galvanic pairs differently configured, measuring potentials, with the help of a reference electrode, and galvanic currents.

In this document, the setting of a set potential or a set current between the electrodes is not envisaged, nor is the use of electrodes with mixed metal oxide coatings, that is, mixed oxides of different metals, in particular noble metals.

Document US1044180 describes galvanic pairs with coatings suitable for low humidity environments for the measurement of oxygen and not of the biofilm. Document US2019/105414 describes the use of strong cathodic reduction, controlled by a potentiostat, to try to eradicate the biofilm on a prothesis.

The present invention as set forth with the appended claims aims to overcome the aforementioned problems by using particular electrochemical biofilm sensors as set forth with claim 1 wherein an embodiment of said sensor comprises:
a first, a second and a third electrode, wherein the first electrode is a working electrode on whose surface the growth of the biofilm to be measured develops, the second electrode is a reference electrode having the function of providing a stable reference potential due to the polarization of the working electrode, the third electrode is a counter-electrode that can be connected to the first electrode through an electrical circuit so as to allow the circulation of current from the first electrode to the third electrode or vice versa, a function of the alteration induced by the biofilm on the kinetics of the evolving reactions on the surface of the first electrode,
being the first electrode and/or the second electrode and/or the third electrode coated with a coating based on mixed oxides of different noble metals, such as iridium, ruthenium, platinum, rhodium, tantalum or niobium,
and wherein
one or more electrodes are made of titanium or tantalum, and
wherein the coating has a thickness between 0.1 and 50 microns, preferably 2.5 microns ± 10%.

The application of the coating usually takes place by immersing the electrode in a solution of salts of the one or more different noble metals mentioned above. Subsequently, in a high temperature atmosphere, the transformation of these salts into ceramic oxides takes place. The final thickness of the coating varies depending on the noble metal load and, usually, is of the order of a few microns.

These coatings, developed since the 1960s, have catalytic properties with regard to different types of reactions, both anodic and cathodic, and are extremely long-lasting and stable over time. Due to the advantages offered, in many areas they have gradually replaced less performing materials, such as graphite. They are currently used in various applications, ranging from the chemical industry to cathodic protection.

Since the aforesaid coatings are substantially not subject to corrosion even in extreme conditions, their application to the aforementioned electrodes makes it possible to extend their life.

In the case of the reference electrode, the proposed innovation also allows for a particularly resistant electrode, with adequately stable potential over time, and economically more advantageous than other solutions on the market.

Finally, as regards the counter-electrode (even if it simultaneously performs the function of "pseudo-reference"), the aforementioned coatings make it possible to advantageously replace materials of the "sacrificial anode" type, thus extending the life of the biofilm sensor, and avoiding pollution by metal ions.

Due to the characteristics listed above, the described coatings can allow the temporary application of suitable and high electric currents, aimed at eliminating (both as a direct consequence of the applied current, and due to oxidizing products generated by electrochemical reactions on the electrode surface) a pre-existing biofilm, thus allowing to carry out an "on-site self-cleaning" of the working electrode and/or of the reference electrode and/or of the counter-electrode, without damaging the electrode being treated. This treatment can be carried out with or without the aid of a service electrode, designed to support the supply of the necessary currents. As previously mentioned, the possibility of self-cleaning one or more electrodes represents a significant advantage, reducing or even completely eliminating the need for further cleaning.

The invention also relates to a device according to claim 5 to measure the growth of a biofilm on the working electrode of a sensor according to one or more of the envisaged embodiments and such a device comprises a potentiostatic circuit equipped with a terminal for the input of a polarization voltage, a feedback circuit to maintain said polarization voltage between the working electrode and the reference/counter-electrode when the working electrode and the reference/counter-electrode of the sensor are connected to the device, a circuit to detect the current flowing between the working electrode and the counter-electrode or alternatively a circuit to detect the voltage present between the working electrode and the reference electrode/counter-electrode, a generator circuit that can be connected manually or by means of switches between pairs of sensor electrodes to temporarily supply a current to clean the electrodes.

According to yet another embodiment, the present invention relates to a system according to claim 8 to monitor the growth of biofilm on a sensor according to one or more of the embodiments described above and such a system comprises a device to measure the growth of a biofilm on the working electrode of a sensor and such a device is connected to the electrodes of a sensor and the system further comprises an ammetric and/or potentiometric circuit driven by a control unit configured to measure over time the current flowing between the working electrode and the counter-electrode and/or the voltage between the working electrode and the reference electrode/counter-electrode with polarized sensor.

Still according to a further embodiment, the invention refers to a process according to claim 11 to apply a coating to the electrodes of a sensor according to one or more of the previous embodiments, such a method comprising the steps of initially immersing the electrode in a solution that includes salts of one or more noble metals such as, for instance, iridium, ruthenium, platinum, niobium, rhodium, tantalum and subsequently subjecting the electrode to high temperature to allow the transformation of the salts into ceramic oxides.

The further characteristics and improvements of the sensor and/or of the device and/or of the system and/or of the process are the subject of the dependent claims. The characteristics of the invention, and the advantages deriving from it, will become more evident from the following detailed description of the attached figures, wherein:
Fig. 1 shows a sensor according to an embodiment of the invention.
Fig. 2 shows the trend as a function of time of the current between the working electrode and the counter-electrode with a biofilm sensor connected to a potentiostat (a) and of the voltage between the reference electrode and the working electrode with a biofilm sensor connected to an intensifier (b) .
Figs. 3 and 4 show examples of connections between electrodes for the detection, respectively, of a voltage or a current as a function of the alteration degree induced by the biofilm on the kinetics of the evolving reactions on the surface of the working electrode of a sensor according to the invention .
Fig. 5 shows an example of a connection that uses a service electrode to clean the working electrode of the sensor according to the invention.
Figs. 6 and 7 show a block diagram of a device to control the electrodes of a sensor according to the invention by monitoring the polarization voltage of the sensor according to the invention.
Figs. 8 and 9 show a block diagram of a device to control the electrodes of a sensor according to the invention by monitoring the current circulating between the working electrode and the counter-electrode of the sensor according to the invention.

To better understand the principle underlying the operation of the sensor of the present invention, the operation of an electrochemical cell is briefly discussed below.

An electrochemical cell normally consists of a reference electrode, a working electrode and a counter-electrode immersed in a saline liquid. By exploiting an appropriate electrical control circuit, it is possible to circulate a current between the working electrode and the counter-electrode, such as to maintain constant the potential set between the working and reference electrode or, on the contrary, measure the potential difference between the working and reference electrode, once the current circulating between the working electrode and the counter-electrode has been set.

When a biofilm is formed on the working electrode, there is an alteration of the oxygen reaction kinetics on this electrode (the depolarization of the cathodic process, which causes an increase in corrosive processes).

The assessment of the rate of oxygen reduction can, therefore, provide a measure of the greater corrosivity induced by the biofilm formation.

On the other hand, the kinetics of the cathodic process can be quantified by determining the value of the current density that goes through an electrode maintained at a predetermined cathodic potential by means of a potentiostat or, equivalently, the value of the potential of an electrode subject to a preset cathodic current flow by means of an intensiostat.

As described in the paper Mollica et al. "On oxygen reduction depolarisation induced by biofilm growth on stainless steel in seawater", European Federation of Corrosion, publication n. 22, 1997, The Institute of Materials, London, p. 51-65 and Faimali et al. "Electrochemical activity and bacterial diversity of natural marine biofilm in laboratory closed-systems, Biochemistry 78, 30-38, 2010, the cathodic current density i (E, t) measured at time t on a stainless steel sample exposed to the action of sea water is, in fact, linked to the cathodic potential set E by the following relationship:
where i1 (E) is the current density measured on the clean fraction of the sample and i2 (E) is the current density measured on the fraction θ (t) of the sample covered by the biofilm (with 0 ≤ θ (t) ≤ 1).

A similar relationship is obtained by making explicit the cathodic potential E as a function of the polarization current density i and the cathodic voltages E1 (i) and E2 (i) measured on clean and biofilm-covered fractions of the sample.

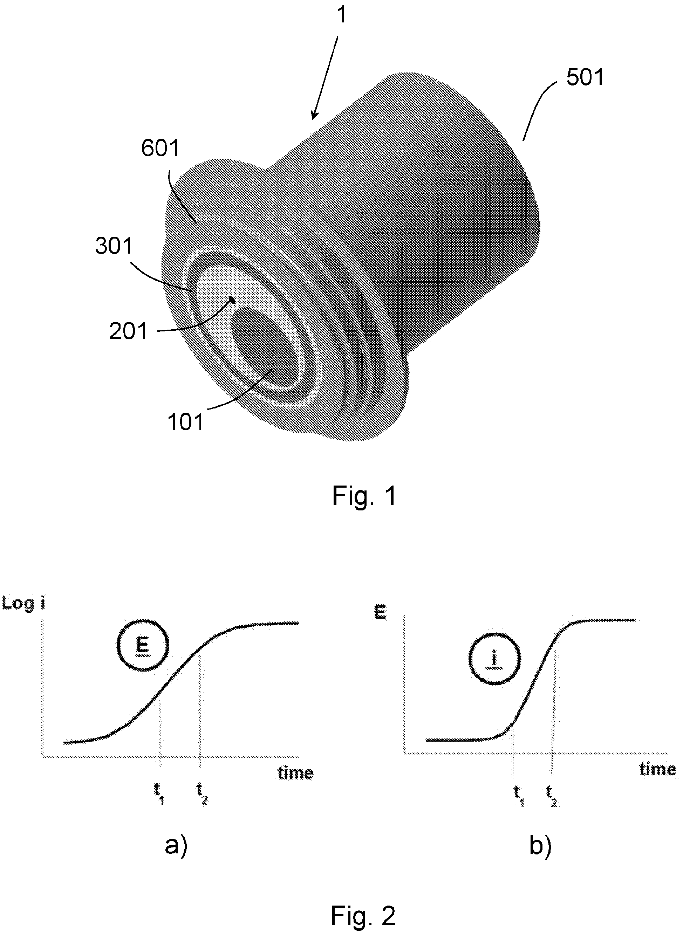

Figures 2a and 2b show, respectively, the typical trend of cathodic current densities i measured at set potential E and potentials E measured with set cathodic current during the growth of a biofilm (Note that with "I" indicates the total current, while "i" the current density).

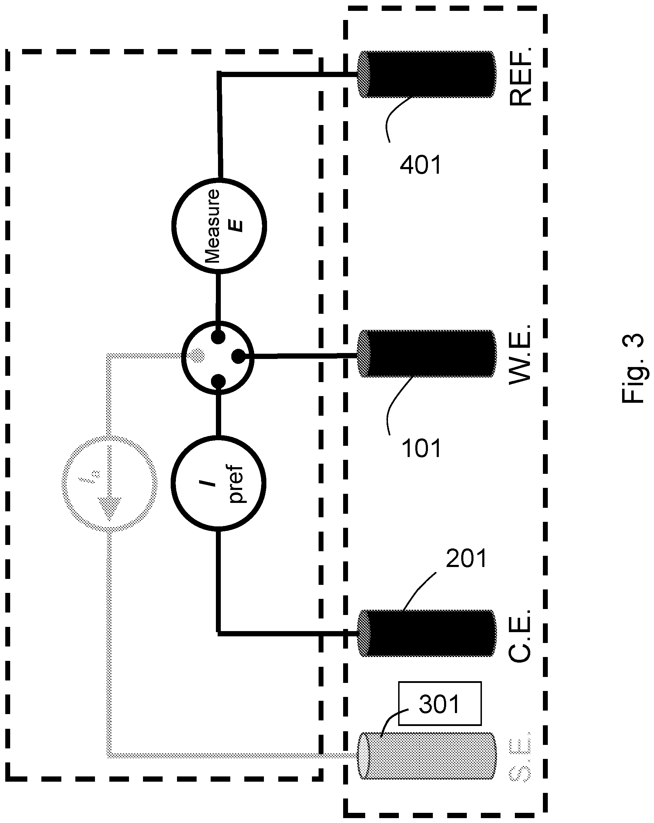

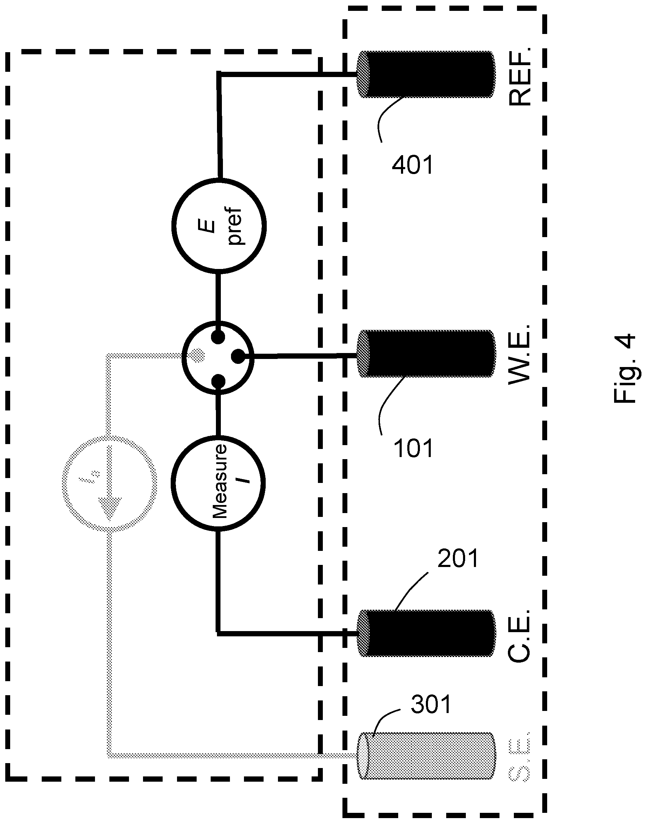

By setting the cathodic polarization current and measuring the cathodic voltage or by setting the polarization voltage E and measuring the cathodic current it is therefore possible to determine the percentage of biofilm that covers a given sample as schematically shown in Fig. 3 and 4.

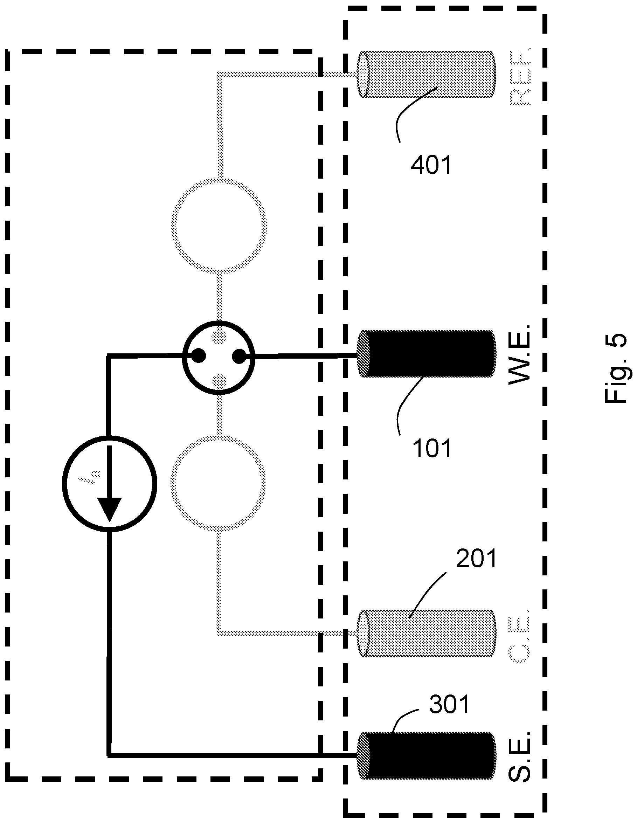

These figures also show a further electrode, called service electrode, which can be connected individually, or in groups, to each of the other electrodes by means of an electrical circuit capable of delivering a current for local self-cleaning of the electrode or electrodes. Fig. 5 shows an example wherein the service electrode is connected to the working electrode.

In an advantageous configuration, the reference electrode and the counter-electrode, indicated separately in the previous figures, can also advantageously form a single electrode having the function of counter-electrode and pseudo reference electrode thus realizing a simpler and more manageable sensor like that of the embodiment shown in Fig. 1.

The sensor 1 shown in this figure comprises, at one end, a working electrode 101, a counter-electrode 201, also serving as a reference electrode, and a service electrode 301 placed side by side with the interposition of insulating elements. In the shown example, each electrode is planar, and connector 601 allows to install the sensor in the process, for instance in a pipeline or in a tank. The planar shape is not limiting and said shape can be modified in relation to the contingent needs of use of the sensor. The end opposite to the one that houses the electrodes contains the electronics necessary to control the sensor and the connector 501 to interface with an external reading and power supply device.

As for the materials, the electrodes are typically made of titanium or tantalum coated with a coating based on mixed metal oxides, typically of noble metals such as iridium, ruthenium, platinum, rhodium, tantalum or niobium.

The coating, which typically has a thickness of between 0.1 and 50 microns, preferably 2.5 microns ± 10%, can be advantageously applied by means of a process which comprises initially immersing the electrode in a solution that includes salts of one or more noble metals such as, for instance, iridium, ruthenium, platinum, niobium, rhodium, tantalum and subsequently subjecting the electrode to high temperature to allow the transformation of salts into ceramic oxides.

The titanium/tantalum support guarantees excellent resistance, good electrical conductivity and can be easily shaped in various forms. The coatings based on mixed metal oxides have catalytic properties with regard to different types of reactions, both anodic and cathodic, and are extremely durable and stable over time as they are scarcely subject to corrosion.

All electrodes can be coated with these oxides independently of each other. That is, there may be a configuration wherein only the working electrode is coated or only the counter-electrode is coated or both are coated as well as situations wherein only the reference electrode is coated or this electrode is coated and the working electrode too is coated and/or the counter-electrode is coated. The same applies to the service electrode which too can be coated like the other electrodes, alone or in one of the previous configurations.

The coatings, due to their characteristic of both electrical and mechanical resistance, can allow the temporary application of suitable and high electrical currents aimed at eliminating (both as a direct consequence of the applied current, and due to oxidizing products generated by electrochemical reactions on the surface of the electrode) a pre-existing biofilm, thus allowing to carry out an "on-site self-cleaning" of the working electrode and/or of the reference electrode and/or of the counter-electrode, without damaging the electrode being treated.

This treatment can be carried out with or without the aid of a service electrode designed to support the supply of the necessary currents.

The ability to self-clean one or more electrodes is a significant advantage that reduces or even completely eliminates the need for maintenance.

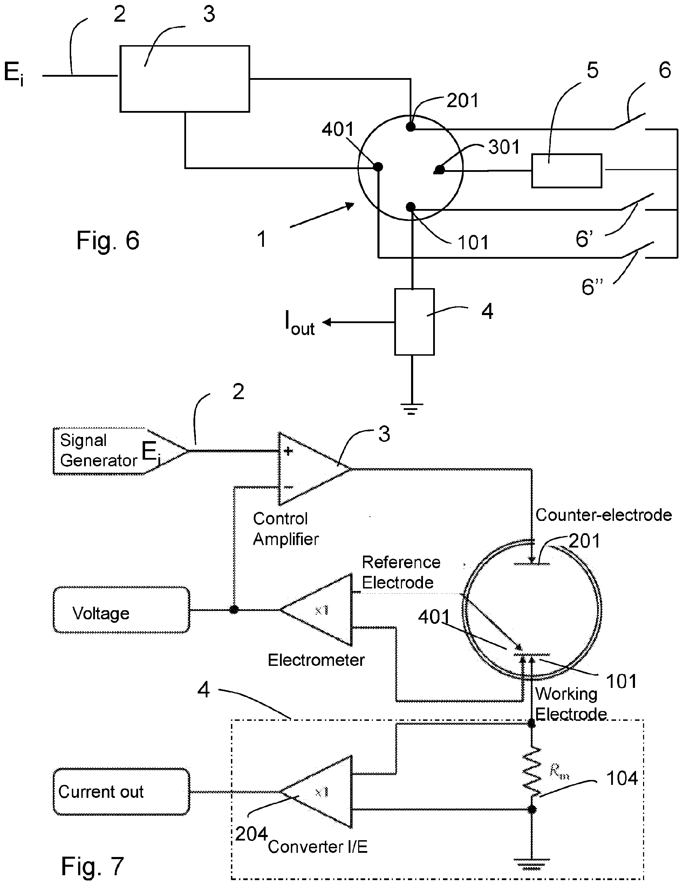

As regards the instrumentation necessary to detect the growth of a biofilm by exploiting the sensors equipped with the electrodes described above, a device with a potentiostatic circuit can be advantageously used. With reference to Fig. 6, the device comprises a terminal 2 for the input of a polarization voltage Ei, a feedback circuit 3 to maintain said polarization voltage between the working electrode 101 and the reference electrode 401, in the case of a configuration with separate counter-electrode and reference electrode, or between the working electrode 101 and the counter-electrode 201, when this too assumes the function of pseudo reference, a detection circuit 4 of the current flowing between the working electrode 101 and counter-electrode 201 and a generator circuit 5 which can be connected manually or by means of switches 6, 6', 6" between pairs of electrodes of the sensor 1 to temporarily supply a current to clean the electrodes.

The feedback circuit 3, which can be operational as shown in Fig. 7, has the task of maintaining the constant polarization voltage between the working electrode 101 and the reference electrode 401.

The current detector circuit 4 can be, for instance, an amplifier 204 which allows to detect the potential differences at the ends of a resistor 104 in series with the working electrode 101 as shown in Fig. 7.

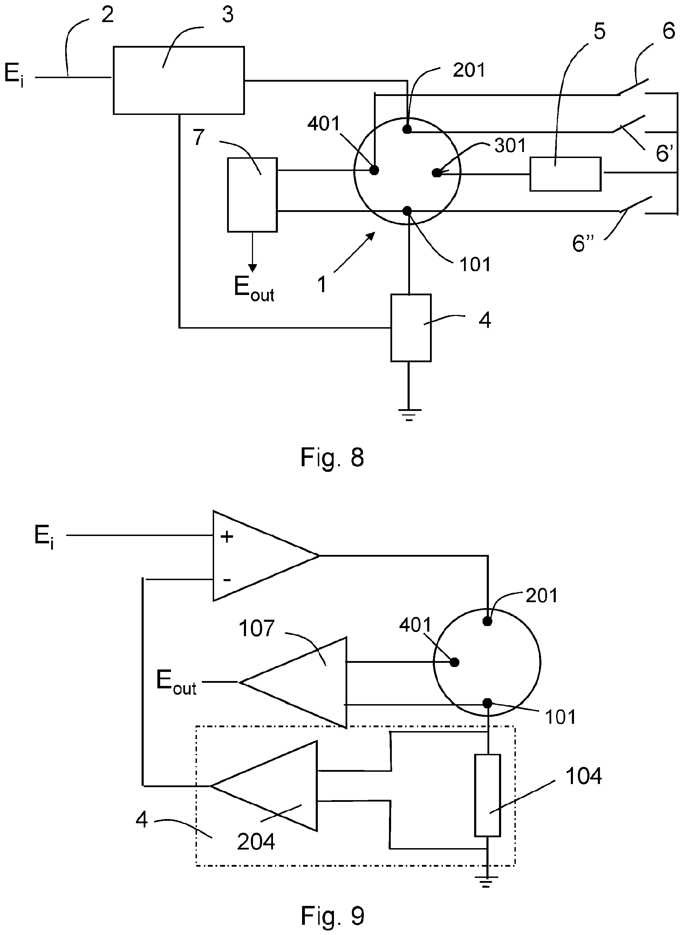

As an alternative to the potentiostatic circuit, an intensiostatic circuit device such as the one shown in Fig. 8 can be used.

In this case the voltage input 2 is used to set a polarization current of the sensor through a feedback circuit 3 to maintain said polarization current between the working electrode 101 and the counter-electrode 201. The current can be detected with a resistor in series 104 and amplifier 204 as indicated in the previous example. The device is completed by a circuit 7, for instance with amplifier 107, to detect the voltage present between the working electrode 101 and the reference electrode 401, in the case of a configuration with separate counter-electrode and reference electrode, or between the working electrode 101 and the counter-electrode 201, when this also assumes the function of pseudo-reference, and a generator circuit 5 which can be connected manually or by means of switches between pairs of electrodes of the sensor to temporarily supply a current to clean the electrodes.

In the configurations shown in Figs. 6 and 8, the generator circuit 5 is advantageously designed to be connected between the service electrode 301 and one or more of the remaining electrodes 101, 201, 401 of the sensor 1 to clean said electrodes.

For this purpose, the generator 5 can be configured to temporarily supply an electric current such as to induce a corresponding current density on the surface of the electrode subject to the cleaning treatment, for instance, in the range of 1 ÷ 100 mA/cm2.

The electrodes and the detection device can be advantageously integrated in the same device or be separate entities.

The detection device can be advantageously interfaced with a control unit to create a biofilm growth monitoring system, for instance on the working electrode of the sensor.

The system advantageously comprises an ammetric and/or potentiometric circuit driven by the control unit to measure over time the current flowing between the working electrode 101 and the counter-electrode 201 and/or the voltage between the working electrode 101 and the reference electrode 401 or the counter-electrode 201 with a polarized sensor according to the type of configuration adopted.

The control unit can also be advantageously configured to act on the generator 5 of the measuring device so as to drive the controlled delivery of a cleaning current between pairs of electrodes of the sensor, in particular between the service electrode 301 and any of the remaining electrodes, for instance by acting on switches 6, 6', 6".

As shown in figures 6 and 8, by means of the switches 6, 6', 6" it is in fact possible to connect the generator 5 to the service electrode 301 and to the working electrode 101 or to the counter-electrode 201 or to the reference electrode 401 so as to make a current flow between the service electrode and the electrode to be cleaned or vice versa.

The invention can be widely modified, for instance by introducing an additional sensing electrode for voltage measurement or by using different configurations for sensor polarization and current and/or voltage measurement whence to derive the area covered by the biofilm. All this without departing from the guiding principle set out above and claimed below.

## Claims

1. An electrochemical biofilm sensor comprising a first (101), a second (401) and a third electrode (201), wherein the first electrode (101) is a working electrode on whose surface the biofilm growth is to be measured, the second electrode (401) is a reference electrode having the function of providing a stable reference potential for the polarization of the working electrode, the third electrode (201) is a counter-electrode connectable to the first electrode (101) by means of an electric circuit so as to allow, at a fixed potential, the circulation of current from the first electrode (101) to the third electrode (201) or vice versa, a function of the alteration induced by the biofilm on the kinetics of the evolving reactions on the surface of the first electrode (101), or so as to allow, at a fixed current, the measurement of the potential of the first electrode (101) with respect to the second electrode (401),
the sensor being **characterized in that**
the first electrode (101) and/or the second electrode (401) and/or the third electrode (201) are coated with a coating based on mixed oxides of different noble metals selected from iridium, ruthenium, platinum, rhodium, tantalum or niobium,
and wherein
one or more electrodes are made of titanium or tantalum, and
wherein the coating has a thickness of between 0.1 and 50 microns, preferably 2.5 microns ± 10%.

2. The sensor according to claim 1, wherein the reference electrode (401) and the counter-electrode (201) form a single electrode having the simultaneous function of counter-electrode and pseudo reference.

3. The sensor according to claim 1 or 2, wherein there is a further service electrode (301) connectable individually, or in groups, to each electrode of the sensor by means of an electric circuit (5) capable of delivering a current for local self-cleaning of the sensor electrode(s).

4. The sensor according to claim 3, wherein the service electrode (301) is coated with a coating based on mixed metal oxides

5. A device to measure the growth of a biofilm on the working electrode (101) of a sensor according to one or more of claims 1 to 4, comprising a potentiostatic circuit equipped with a terminal (2) for the input of a polarization voltage, a feedback circuit (3) to maintain said polarization voltage between the working electrode (101) and the reference/counter-electrode (401, 201) when the working electrode (101) and the reference/counter-electrode of the sensor (401, 201) are connected to the device, a detection circuit (4) of the current flowing between the working electrode (101) and the counter-electrode (201) or alternatively a detection circuit (7) of the voltage between the working electrode (101) and the reference electrode/counter-electrode (401, 201), a generator circuit (5) that can be connected manually or by means of switches (6, 6', 6") between pairs of electrodes (101, 201, 401) of the sensor to temporarily draw a current to clean the electrodes.

6. The device according to claim 5, wherein the generator circuit (5) is provided to be connected between the service electrode (301) and one or more of the remaining electrodes (101, 201, 401) of the sensor to clean said electrodes.

7. The device according to claim 5 or 6, wherein the generator (5) is configured for the temporary supply of an electric current such as to induce a corresponding current density on the surface of the electrode subject to the cleaning treatment comprised in the range 1 ÷ 100 mA/cm2.

8. A system to monitor the growth of biofilm on a sensor according to one or more of claims 1 to 4, comprising a device according to one or more of claims 5 to 7 connected to the electrodes of a sensor according to one or more of claims 1 to 4 , the system comprising an ammetric (4) and/or potentiometric (7) circuit driven by a control unit configured to measure over time the current flowing between the working electrode (101) and the counter-electrode (201) and/or the voltage between the working electrode (101) and the reference/counter-electrode (401, 201) with a polarized sensor.

9. The system according to claim 8, wherein the control unit is configured to act on the generator (5) of the measuring device so as to drive the controlled delivery of a cleaning current between pairs of sensor electrodes.

10. The system according to claim 9, wherein there are switches (6, 6', 6") that can be controlled by the control unit to connect the generator to the service electrode (301) and to the working electrode (101) either to the counter-electrode (201) or to the reference electrode (401) so as to make a current flow between the service electrode (301) and the electrode to be cleaned or vice versa.

11. A process to apply a coating to the electrodes of a sensor according to one or more of the preceding claims 1 to 4,
**characterized by** the fact of initially immersing the electrode in a solution that includes salts of more than one noble metals selected from iridium, ruthenium, platinum, niobium, rhodium, tantalum and subsequently subjecting the electrode to high temperature to allow the transformation of salts into ceramic oxides.

## Patentansprüche

1. Elektrochemischer Biofilmsensor umfassend eine erste (101), eine zweite (401) und eine dritte Elektrode (201), wobei die erste Elektrode (101) eine Arbeitselektrode ist, auf deren Oberfläche das Biofilmwachstum zu messen ist, und die zweite Elektrode (401) eine Referenzelektrode ist, die die Funktion hat, ein stabiles Referenzpotential für die Polarisation der Arbeitselektrode bereitzustellen, die dritte Elektrode (201) eine Gegenelektrode ist, die mit der ersten Elektrode (101) über einen Stromkreis derart verbunden werden kann, dass sie bei einem festen Potential den Stromkreislauf von der ersten Elektrode (101) zur dritten Elektrode (201) oder umgekehrt ermöglicht, in Abhängigkeit von der durch den Biofilm induzierten Veränderung der Kinetik der sich auf der Oberfläche der ersten Elektrode (101) vollziehenden Reaktionen zu ermöglichen, oder um bei einem festen Strom die Messung des Potentials der ersten Elektrode (101) in Bezug auf die zweite Elektrode (401) zu ermöglichen, wobei der Sensor **dadurch gekennzeichnet ist, dass** die erste Elektrode (101) und/oder die zweite Elektrode (401) und/oder die dritte Elektrode (201) mit einer Beschichtung auf Basis von gemischten Oxiden von verschiedenen Edelmetallen beschichtet sind, die aus Iridium, Ruthenium, Platin, Rhodium, Tantal oder Niob ausgewählt sind, und wobei eine oder mehrere Elektroden aus Titan oder Tantal hergestellt sind, und wobei die Dicke der Beschichtung zwischen 0,1 und 50 Mikrometer, vorzugsweise 2,5 Mikrometer ± 10 % beträgt.

2. Sensor nach Anspruch 1, wobei die Referenzelektrode (401) und die Gegenelektrode (201) eine einzige Elektrode bilden, die gleichzeitig die Funktion von Gegenelektrode und Pseudoreferenz hat.

3. Sensor nach Anspruch 1 oder 2, wobei eine weitere Betriebselektrode (301) vorhanden ist, die einzeln oder in Gruppen mit jeder Elektrode des Sensors mittels einer elektrischen Schaltung (5) anschließbar ist und in der Lage ist, einen Strom zur lokalen Selbstreinigung der Sensorelektrode(n) bereitzustellen.

4. Sensor nach Anspruch 3, wobei die Betriebselektrode (301) mit einer Beschichtung auf Basis von gemischten Metalloxiden beschichtet ist.

5. Vorrichtung zur Messung des Wachstums eines Biofilms auf der Arbeitselektrode (101) eines Sensors nach einem oder mehreren der Ansprüche 1 bis 4, umfassend eine potentiostatische Schaltung, die mit einem Anschluss (2) zur Eingabe einer Polarisationsspannung ausgestattet ist, eine Rückkopplungsschaltung (3) zur Aufrechterhaltung der Polarisationsspannung zwischen der Arbeitselektrode (101) und der Referenz-/Gegenelektrode (401, 201), wenn die Arbeitselektrode (101) und die Referenz-/Gegenelektrode des Sensors (401, 201) mit der Vorrichtung verbunden sind, eine Schaltung (4) zur Erfassung des zwischen der Arbeitselektrode (101) und der Gegenelektrode (201) fließenden Stroms oder alternativ eine Schaltung (7) zur Erfassung der Spannung zwischen der Arbeitselektrode (101) und der Referenzelektrode/Gegenelektrode (401, 201), eine Generatorschaltung (5), die manuell oder über Schalter (6, 6', 6") zwischen Elektrodenpaaren (101, 201, 401) des Sensors angeschlossen werden kann, um vorübergehend einen Strom zur Reinigung der Elektroden zu liefern.

6. Vorrichtung nach Anspruch 5, wobei die Generatorschaltung (5) dazu vorgesehen ist, zwischen der Betriebselektrode (301) und einer oder mehreren der übrigen Elektroden (101, 201, 401) des Sensors angeschlossen zu werden, um diese Elektroden zu reinigen.

7. Vorrichtung nach Anspruch 5 oder 6, wobei der Generator (5) für die vorübergehende Bereitstellung eines elektrischen Stroms derart konfiguriert ist, dass er auf der Oberfläche der der Reinigungsbehandlung unterzogenen Elektrode eine entsprechende Stromdichte im Bereich von 1 ÷ 100 mA/cm² induziert.

8. System zur Überwachung des Wachstums von Biofilm auf einem Sensor nach einem oder mehreren der Ansprüche 1 bis 4, umfassend eine Vorrichtung nach einem oder mehreren der Ansprüche 5 bis 7, die mit den Elektroden eines Sensors nach einem oder mehreren der Ansprüche 1 bis 4 verbunden ist, wobei das System eine amperometrische (4) und/oder potentiometrische (7) Schaltung umfasst, die von einer Steuereinheit gesteuert wird, welche dazu konfiguriert ist, den zwischen der Arbeitselektrode (101) und der Gegenelektrode (201) fließenden Strom und/oder die Spannung zwischen der Arbeitselektrode (101) und der Referenz-/Gegenelektrode (401, 201) mit einem polarisierten Sensor über die Zeit zu messen.

9. System nach Anspruch 8, wobei die Steuereinheit derart eingerichtet ist, dass sie auf den Generator (5) des Messgeräts zur gesteuerten Abgabe eines Reinigungsstroms zwischen Sensorelektrodenpaaren einwirkt.

10. System nach Anspruch 9, wobei Schalter (6, 6', 6") vorgesehen sind, die von der Steuereinheit gesteuert werden können, um den Generator mit der Betriebselektrode (301) und mit der Arbeitselektrode (101) oder mit der Gegenelektrode (201) oder mit der Referenzelektrode (401) zu verbinden, um einen Stromfluss zwischen der Betriebselektrode (301) und der zu reinigenden Elektrode oder umgekehrt zu erzeugen.

11. Verfahren zum Auftragen einer Beschichtung auf die Elektroden eines Sensors nach einem oder mehreren der vorhergehenden Ansprüche 1 bis 4, **dadurch gekennzeichnet, dass** die Elektrode zunächst in eine Lösung getaucht wird, die Salze von einem oder mehreren Edelmetallen, ausgewählt aus Iridium, Ruthenium, Platin, Niob, Rhodium und Tantal, enthält, und dass die Elektrode anschließend einer hohen Temperatur ausgesetzt wird, um die Umwandlung der Salze in keramische Oxide zu ermöglichen.

## Revendications

1. Capteur électrochimique de biofilm comprenant une première (101), une deuxième (401) et une troisième électrode (201), dans lequel la première électrode (101) est une électrode de travail sur la surface de laquelle doit être mesurée la croissance du biofilm, la deuxième électrode (401) est une électrode de référence ayant pour fonction de fournir un potentiel de référence stable pour la polarisation de l'électrode de travail, la troisième électrode (201) est une contre-électrode pouvant être connectée à la première électrode (101) au moyen d'un circuit électrique de manière à permettre, à un potentiel fixé, la circulation de courant de la première électrode (101) à la troisième électrode (201) ou vice versa, en fonction de l'altération induite par le biofilm sur la cinétique des réactions se développant à la surface de la première électrode (101), ou de manière à permettre, à un courant fixé, la mesure du potentiel de la première électrode (101) par rapport à la deuxième électrode (401),
le capteur étant **caractérisé en ce que**
la première électrode (101) et/ou la deuxième électrode (401) et/ou la troisième électrode (201) sont recouvertes d'un revêtement à base d'oxydes mixtes de différents métaux nobles choisis parmi iridium, ruthénium, platine, rhodium, tantale ou niobium,
et, où
une ou plusieurs électrodes sont en titane ou en tantale, et
où le revêtement a une épaisseur comprise entre 0,1 et 50 microns, de préférence 2,5 microns ± 10 %.

2. Le capteur selon la revendication 1, dans lequel l'électrode de référence (401) et la contre-électrode (201) forment une seule électrode ayant la fonction simultanée de contre-électrode et de pseudo-référence.

3. Le capteur selon la revendication 1 ou 2, dans lequel il y a une électrode de service (301) supplémentaire qui peut être connectée individuellement, ou en groupes, à chaque électrode du capteur au moyen d'un circuit électrique (5) capable de distribuer un courant pour l'auto-nettoyage local de l'électrode ou des électrodes du capteur.

4. Le capteur selon la revendication 3, dans lequel l'électrode de service (301) est recouverte d'un revêtement à base d'oxydes métalliques mixtes.

5. Un dispositif pour mesurer la croissance d'un biofilm sur l'électrode de travail (101) d'un capteur selon une ou plusieurs des revendications de 1 à 4, comprenant un circuit potentiostatique équipé d'une borne (2) pour l'entrée d'une tension de polarisation, un circuit de rétroaction (3) pour maintenir ladite tension de polarisation entre l'électrode de travail (101) et l'électrode de référence/contre-électrode (401, 201) lorsque l'électrode de travail (101) et l'électrode de référence/contre-électrode (401, 201) du capteur sont connectées au dispositif, un circuit de détection (4) du courant qui circule entre l'électrode de travail (101) et la contre-électrode (201) ou bien un circuit de détection (7) de la tension entre l'électrode de travail (101) et l'électrode de référence/contre-électrode (401, 201), un circuit générateur (5) qui peut être connecté manuellement ou au moyen d'interrupteurs (6, 6', 6") entre des paires d'électrodes (101, 201, 401) du capteur pour prélever temporairement un courant afin de nettoyer les électrodes.

6. Le dispositif selon la revendication 5, dans lequel le circuit générateur (5) est prévu pour être connecté entre l'électrode de service (301) et une ou plusieurs des électrodes restantes (101, 201, 401) du capteur pour nettoyer lesdites électrodes.

7. Le dispositif selon la revendication 5 ou 6, dans lequel le générateur (5) est configuré pour l'alimentation temporaire d'un courant électrique de manière à induire, sur la surface de l'électrode soumise au traitement de nettoyage, une densité de courant correspondante comprise dans la plage 1÷100 mA/cm².

8. Un système de surveillance de la croissance d'un biofilm sur un capteur selon l'une ou plusieurs des revendications de 1 à 4, comprenant un dispositif selon une ou plusieurs des revendications de 5 à 7 connecté aux électrodes d'un capteur selon une ou plusieurs des revendications de 1 à 4, le système comprenant un circuit ampérométrique (4) et/ou potentiométrique (7) piloté par une unité de commande configurée pour mesurer au cours du temps le courant circulant entre l'électrode de travail (101) et la contre-électrode (201) et/ou la tension entre l'électrode de travail (101) et l'électrode de référence/contre-électrode (401, 201) avec un capteur polarisé.

9. Le système selon la revendication 8, dans lequel l'unité de commande est configurée pour agir sur le générateur (5) du dispositif de mesure de manière à piloter la distribution contrôlée d'un courant de nettoyage entre paires d'électrodes du capteur.

10. Le système selon la revendication 9, dans lequel sont présents des interrupteurs (6, 6', 6") qui peuvent être commandés par l'unité de commande pour connecter le générateur à l'électrode de service (301) et à l'électrode de travail (101), ou à la contre-électrode (201) ou à l'électrode de référence (401), de manière à faire circuler un courant entre l'électrode de service (301) et l'électrode à nettoyer, ou vice versa.

11. Un procédé pour appliquer un revêtement sur les électrodes d'un capteur selon une ou plusieurs des revendications précédentes de 1 à 4,
**caractérisé en ce qu'**on va immerger initialement l'électrode dans une solution qui comprend des sels de plus d'un métal noble choisi parmi iridium, ruthénium, platine, niobium, rhodium, tantale et ensuite on va soumettre l'électrode à une température élevée pour permettre la transformation des sels en oxydes céramiques.
